# EUROPÄISCHE PATENTSCHRIFT

(11) **EP 3 558 152 B1**
(45) Veröffentlichungstag und Bekanntmachung des Hinweises auf die Patenterteilung: **27.01.2021**
(21) Anmeldenummer: 17826173.1
(22) Anmeldetag: 15.12.2017
(51) Int. Cl.: A61B 46/20

(54) **OPERATIONSABDECKUNG**
SURGICAL DRAPE
STRUCTURE DE RECOUVREMENT POUR OPÉRATIONS

(30) Priorität: 22.12.2016 DE 102016125359
(43) Veröffentlichungstag der Anmeldung: 30.10.2019
(73) Patentinhaber: Paul Hartmann AG, 89522 Heidenheim (DE)
(72) Erfinder: HOFFMANN, Thomas, 89522 Heidenheim (DE); LEISTENSCHNEIDER, Denise, 89429 Bachhagel (DE); LINDNER, Leonie, 40625 Düsseldorf (DE)
(86) Internationale Anmeldenummer: PCT/EP2017/082982
(87) Internationale Veröffentlichungsnummer: WO 2018/114647

(56) Entgegenhaltungen:
- EP-A2- 0 051 935
- WO-A1-2015/014781
- WO-A2-2008/141137
- DE-A1-102007 022 878

## Beschreibung

Die vorliegende Erfindung betrifft eine Operationsabdeckung zur zumindest bereichsweisen Abdeckung eines menschlichen oder tierischen Körpers, mit einer Oberseite und einer Unterseite, mit einem Basistuchelement und mit zumindest einer innerhalb des Basistuchelements angeordneten Öffnung, wobei diese Öffnung von einem Folienelement überfangen ist und wobei dieses Folienelement eine erste Schwächungslinie aufweist und wobei die erste Schwächungslinie im Bereich der Öffnung angeordnet ist. Das Folienelement weist dabei auf der Unterseite zumindest innerhalb der Öffnung einen klebenden Bereich, insbesondere eine klebende Schicht auf und auf dem klebenden Bereich, insbesondere auf der klebenden Schicht, ist ein ablösbares Schutzmaterialelement angeordnet, wobei das Schutzmaterialelement eine zweite Schwächungslinie aufweist.

Operationsabdeckungen, deren Öffnungen im Basistuchelement mit einem Folienelement überdeckt sind, wobei das Folienelement einen klebenden Bereich, insbesondere eine klebende Schicht aufweist, werden eingesetzt, um das spätere Eingriffsfeld möglichst keimfrei abdichten zu können. Zur Vermeidung des Einsatzes von mechanischen Schneidegeräten ist es bekannt, eine Schwächungslinie im Folienelement vorzusehen. Die für die Operation, auf den Patienten und dessen anatomische Gegebenheiten abgestimmte Zugriffstelle durch das Folienelement kann dann bedarfsgerecht durch das Öffnen entlang der Schwächungslinie freigelegt werden. Der klebende Bereich bzw. die klebende Schicht wird bis zur Anwendung der Operationsabdeckung herkömmlich mit einem Schutzmaterialelement, wie beispielsweise einem Release-Papier abgedeckt.

Derartige Operationsabdeckungen sind aus dem Stand der Technik bekannt, wie beispielweise aus dem Dokument DE 10 2007 022 878 B4.

In DE 10 2007 022 878 B4 ist weiter vorgeschlagen, die Schlitze bzw. Sollbruchstellen des flexiblen Flächenelements, insbesondere der Folie, deckungsgleich mit Schlitzen bzw. Sollbruchstellen in der dem flexiblen Flächenelement zugeordneten Schutzfolie bereitzustellen. Damit werden eine einfache Herstellbarkeit und eine universelle Verwendbarkeit des Operationstuches erreicht.

Das Positionieren, Drapieren und Fixieren einer Operationsabdeckung an den Patienten gestaltet sich hingegen oftmals schwierig. Das Folienelement ist zumeist aus dünnem und flexiblem Material und damit schwierig handhabbar. Bei einer herstellerseitig im Folienelement eingebrachten Schwächungslinie können bereits beim Entfernen des Schutzmaterials von der klebenden Seite des Folienelements unerwünschte und unkontrollierte Öffnungsvorgänge des Folienelements entlang der Schwächungslinie eingeleitet werden. Bis zum Fixieren des Folienelements an den Patienten kann es zu unerwünschten Überfältelungen an den geöffneten Abschnitten der Schwächungslinie und damit womöglich zu ungewollten Verklebungen innerhalb des Folienelements kommen.

Ausgehend von dem bekannten Stand der Technik liegt nun der Erfindung die Aufgabe zugrunde, den vorstehend geschilderten Problemen zu begegnen.

Diese Aufgabe wird gelöst durch eine Operationsabdeckung mit den Merkmalen des Anspruchs 1.

Dabei ist vorgesehen, dass das Schutzmaterialelement derart auf dem Folienelement angeordnet ist, dass die zweite Schwächungslinie des Schutzmaterialelements die erste Schwächungslinie des Folienelements kreuzt.

Es wurde erkannt, dass bei einem Schutzmaterialelement mit einer darin eingebrachten Schwächungslinie, das Schutzmaterialelement typischerweise um eine zu seiner Schwächungslinie parallel verlaufenden Achse oder direkt um eine in der Schwächungslinie verlaufenden Achse vom Folienelement abgezogen wird. Bei einer deckungsgleichen Anordnung der Schwächungslinien von Schutzmaterialelement und von Folienelement wird damit beim Entfernen des Schutzmaterialelements die Schwächungslinie des Folienelements in ihrer gesamten Länge freigesetzt und auch der an die Schwächungslinie angrenzende Folienelementabschnitt mit seiner gesamten Fläche. Es besteht dabei die Gefahr, dass die beim Entfernen des Schutzmaterialelements auf das Schutzmaterialelement ausgeübten Zug- oder Peelkräfte unmittelbar auf das Folienelement übertragen werden, derart, dass solche Kräfte unmittelbar auf die Schwächungslinie des Folienelements einwirken und somit ein unvermitteltes Öffnen zumindest entlang von Teilabschnitten der Schwächungslinie herbeiführen.

Mit der vorliegenden Erfindung wurde erkannt, dass bei einer Anordnung des Folienelements mit einer ersten Schwächungslinie und des Schutzmaterialelements mit einer zweiten Schwächungslinie, derart, dass sich erste und zweite Schwächungslinie kreuzen, also nicht deckungsgleich übereinander liegen, das Entfernen des Schutzmaterialelements anwendungsfreundlicher möglich ist: Durch das mögliche Umfalten des Schutzmaterialelements an einer Achse auf sich selbst, insbesondere an einer Achse parallel zu der zweiten Schwächungslinie, insbesondere entlang der zweiten Schwächungslinie des Schutzmaterialelements, wird das Folienelement und damit die erste Schwächungslinie lediglich sukzessive freigelegt. Beim Entfernen des Schutzmaterialelements verteilen sich die dabei auftretenden Zug- oder Peelkräfte größtenteils auf ungeschwächtes Folienelementmaterial, so dass die erste Schwächungslinie des Folienelementmaterials vor ungewollt vorzeitigem Bruch besser geschützt ist. Ebenso wurde erkannt, dass ein Schutzmaterialelement mit einer solchen Anordnung der zweiten Schwächungslinie vorteilhaft als Applikations- und Griffhilfe genutzt werden kann.

Die Möglichkeit des sukzessiven Entfernens des Schutzmaterialelements vom Folienelement, ist insbesondere von besonderem Vorteil, wenn das Schutzmaterialelement im Vergleich zum Folienelement ein dickeres, stärkeres und/oder steiferes Material aufweist.

Im Rahmen dieser Erfindung wird eine "Schwächungslinie" als eine das Folienelement und/oder das Schutzmaterialelement in seiner Unversehrtheit ändernde Linie verstanden. Eine Schwächungslinie kann dabei eine Linie mit ausschließlich nur partiell über die Dicke des Flachbahnmaterials eingebrachten Deformationsbereiche ausgeführt sein. Eine Schwächungslinie kann als eine Falzachse ausgeführt sein. Eine Schwächungslinie kann auch als eine alternierende Anordnung von ein Flachbahnmaterial trennenden Bereichen und das Flachbahnmaterial verbindenden Stegen ausgeführt sein. Dabei können die trennenden Bereiche und die verbindenden Stege (Verbindungsstege) unterschiedlich ausgebildet sein. Die trennenden Bereiche können dabei beispielsweise als geradlinige, gekrümmte oder gewellte Schnitte, als Löcher, als eingestanzte und damit im Material teilweise geschwächte Abschnitte - so in Form von gequetschten Stellen ausgestaltet sein. Die Verbindungsstege können ebenso verschieden gestaltet sein, so vorzugsweise als unmittelbare und unbeschädigte Materialbrücken. Durch die Materialbrücken bzw. die Verbindungsstege bleibt im Gegensatz zu einer durchgehenden Schnittlinie zumindest eine gewisse Zusammengehörigkeit des jeweiligen Flachmaterials erhalten, d. h. die Verbindungsstege verhindern einen unerwünschten sofortigen, unvermittelten Öffnungsvorgang.

Mit "Oberseite" wird eine Seite der Operationsabdeckung oder einer der Lagen der Operationsabdeckung verstanden, die im Anwendungszustand vom Patienten abgewandt ist. Mit "Unterseite" wird die andere, im Anwendungszustand dem Patienten zugewandte Seite der Operationsabdeckung oder einer der Lagen der Operationsabdeckung verstanden.

Vorteilhafte Weiterbildungen der Operationsabdeckung ergeben sich aus den nachfolgend beschriebenen Ausführungsformen und den jeweiligen Unteransprüchen.

In einer bevorzugten Ausführungsform der Operationsabdeckung weist das Folienelement nur eine einzige erste Schwächungslinie und das Schutzmaterialelement nur eine einzige zweite Schwächungslinie auf.

Es kann vorzugsweise jedoch auch vorgesehen sein, dass das Folienelement eine weitere erste Schwächungslinie und/oder das Schutzmaterialelement zumindest eine weitere, insbesondere zwei weitere oder mehrere weitere zweite Schwächungslinien aufweisen.

Die mögliche weitere erste und/oder weitere zweite Schwächungslinie ist vorzugsweise ebenso in einer erfindungsgemäßen, sich kreuzenden Anordnung der beiden Schwächungslinien zueinander vorgesehen.

In einer bevorzugten Ausführungsform der Operationsdeckung weist insbesondere das Schutzmaterialelement eine, zwei oder mehrere weitere zweite Schwächungslinien auf, während das Folienelement nur eine einzige erste Schwächungslinie aufweist.

Mit mehreren zweiten Schwächungslinien im Schutzmaterialelement können damit vorteilhaft mehrere Achsen zum bereichsweisen Umfalten und/oder Entfernen des Schutzmaterialelements bereitgestellt werden.

Vorzugsweise weist das Folienelement einen ersten Folienelementabschnitt und einen zweiten Folienelementabschnitt auf, wobei der erste Folienelementabschnitt entlang der ersten Schwächungslinie von dem zweiten Folienelementabschnitt trennbar ist.

Mit der Trennbarkeit der Folienelementabschnitte voneinander wird vorteilhaft eine Portionierung des Folienelements und damit eine an den Patienten und dessen anatomischen Gegebenheiten und/oder eine den Operationsbedingungen angepasste bereichsweise Andrapierung und Fixierung des Folienelements ermöglicht, ohne hierzu weitere mechanische Hilfsmittel, wie Schere oder Skalpell heranziehen zu müssen.

Alternativ oder vorzugsweise zusätzlich kann das Schutzmaterialelement einen ersten Schutzmaterialelementabschnitt und einen zweiten Schutzmaterialelementabschnitt aufweisen, wobei der erste Schutzmaterialelementabschnitt entlang der zweiten Schwächungslinie um den zweiten Schutzmaterialelementabschnitt faltbar und/oder insbesondere von dem zweiten Schutzmaterialelementabschnitt trennbar ist. In denkbaren Ausführungen mit zumindest einer weiteren zweiten Schwächungslinie sind die durch diese zweiten Schwächungslinien voneinander abgegrenzten Schutzmaterialelementabschnitte entsprechend um die durch die Schwächungslinien beschriebenen Achsen faltbar und/oder trennbar.

Durch die Faltung des Schutzmaterialelements entlang der zweiten Schwächungslinie kann dem Anwender vorteilhaft eine Applikationshilfe an die Hand gegeben werden.

Insbesondere vorteilhaft ist die zweite oder weitere zweite Schwächungslinie derart ausgebildet, dass sich die zweite Schwächungslinie nicht vollständig bis in Randbereiche des Schutzmaterialelements erstreckt, sondern das Schutzmaterialelement in den Randbereichen intakt verbleibt. Eine leichte Abtrennbarkeit der Schutzmaterialelementabschnitte voneinander wird zwar dadurch unterbunden, jedoch um vorteilhaft eine zusammenhängende Applikationshilfe und auch damit eine Grifflasche für den fortlaufenden Abziehvorgang des Schutzmaterialelements bereitzustellen. Damit wird auch die Freisetzung von mehreren losen Schutzmaterialelementabschnitten, die nicht im sterilen OP-Feld verbleiben dürfen und der Entsorgung zugeführt werden müssen, vermieden.

Alternativ sind jedoch auch Ausführungsformen denkbar, in denen der erste Schutzmaterialelementabschnitt entlang der zweiten oder weiteren zweiten Schwächungslinie von dem zweiten oder weiteren Schutzmaterialabschnitt trennbar ist.

Insbesondere bevorzugt weist das Schutzmaterialelement einen ersten Schutzmaterialelementabschnitt und einen zweiten Schutzmaterialelementabschnitt auf, wobei der erste Schutzmaterialelementabschnitt entlang der zweiten Schwächungslinie in Richtung auf den zweiten Schutzmaterialelementabschnitt faltbar ist und der erste Schutzmaterialelementabschnitt in dieser Konfiguration als eine Applikationshilfe, insbesondere als ein Anfassmittel nutzbar ist.

Weiter vorzugsweise weist die Operationsabdeckung eine Längsrichtung und eine Querrichtung auf, wobei die erste Schwächungslinie zumindest bereichsweise, insbesondere ausschließlich in Querrichtung oder Längsrichtung verläuft und/oder die zweite Schwächungslinie zumindest bereichsweise, insbesondere ausschließlich in der jeweils anderen Längsrichtung oder Querrichtung verläuft.

Der Verlauf der ersten und/oder zweiten Schwächungslinie in Längsrichtung und/oder Querrichtung der Operationsabdeckung erleichtert dem Anwender die Anwendung der Operationsabdeckung, da die Schwächungslinien innerhalb der Operationsabdeckung im Sinne einer leicht erkennbaren Koordinate angeordnet sind, und der Anwender vor allem in zeitkritischen Notfallsituationen schneller handeln kann.

Als "Längsrichtung" wird dabei jegliche Erstreckung zwischen zwei sich gegenüber liegenden Enden, insbesondere einem oberen und einem unteren Ende der Operationsabdeckung verstanden. Das obere und untere Ende der Operationsabdeckung wird durch die im Gebrauch der Operationsabdeckung vorgesehene Anordnung in Relation zur Bodenebene festgelegt. Ein oberes und ein unteres Ende in Längsrichtung der Operationsabdeckung werden durch den vorgesehenen Einsatzzweck der jeweiligen Operationsabdeckung bestimmt. Die "Querrichtung" ist die Richtung senkrecht zur Längsrichtung.

Die für den jeweiligen Einsatzzweck bevorzugte Anordnung der Operationsabdeckung in Längsrichtung bzw. Querrichtung wird dem Anwender, also dem medizinischen Personal, vorteilhafterweise durch Orientierungshilfen angezeigt. Als Orientierungshilfen sind dabei verschiedene Ausführungen denkbar, so beispielsweise in Form von Symbolen, Grafiken, Pfeilen mit Richtungsangabe oder auch als schematische Darstellung von Mensch oder Tier, von Körperteilen wie beispielsweise Kopf, welche dem Anwender die Orientierung von einem oberen und unteren Ende der Operationsabdeckung suggerieren.

Die Orientierungshilfen sind vorzugweise auf einer Sichtseite des Schutzmaterialelements oder in der Nähe zum Schutzmaterialelement auf dem Basistuchelement, insbesondere auf der Unterseite des Basistuchelements angebracht.

In einer vorteilhaften Ausführung kann vorgesehen sein, dass die zweite Schwächungslinie einen an der ersten Schwächungslinie achsensymmetrisch gespiegelten Verlauf aufweist.

Durch einen achsenspiegelsymmetrischen Verlauf der zweiten Schwächungslinie kann bei der Handhabung des Schutzmaterialelements, so eben beim Um- bzw. Zurückfalten des Schutzmaterialelements und/oder beim Entfernen vom Folienelement ein identischer Kräfteverlauf beidseits der ersten Schwächungslinie auf die Folienelementabschnitte als auch auf die erste Schwächungslinie selbst ermöglicht werden, was einem gelenkten Öffnen der ersten Schwächungslinie zuträglich ist.

Alternativ sind auch Anordnungen der Schwächungslinien zueinander denkbar, in denen die zweite Schwächungslinie einen in einem Kreuzungspunkt mit der ersten Schwächungslinie punktsymmetrisch gespiegelten Verlauf beibehält.

Vorzugsweise kreuzt die zweite Schwächungslinie die erste Schwächungslinie ausgehend von einem der Enden der ersten Schwächungslinie in einem Winkel α von vorzugsweise 45° - 90 °. In einer besonders bevorzugten Ausführung kreuzt die zweite Schwächungslinie die erste Schwächungslinie in einem Winkel α von 90°, also die zweite und erste Schwächungslinie stehen in einer senkrechten Anordnung zueinander.

Zudem kann bei Ausführungsformen mit einem Winkel α kleiner als 90°, der Verlauf, insbesondere ein visuell erkennbarer oder markierter Verlauf der zweiten Schwächungslinie als Orientierungshilfe für den Anwender, insbesondere als Orientierungshilfe für eine bevorzugte Abzugs- und/oder Umfaltrichtung des Schutzmaterialelements dienen.

Im Falle, dass die zweite Schwächungslinie die erste Schwächungslinie in einem von einem geraden Verlauf abweichenden Verlauf kreuzt, wie eben im Falle einer Krümmung, so ist im Kreuzungspunkt der ersten und zweiten Schwächungslinie eine Tangente an die Schwächungslinien anzulegen und der aus der Kreuzung dieser Tangenten ermittelte Winkel ist als Winkel α heranzuziehen.

Insbesondere ist vorgesehen, dass die zweite Schwächungslinie die erste Schwächungslinie derart in einem Kreuzungspunkt kreuzt, dass der Kreuzungspunkt zumindest innerhalb eines ersten Viertels, insbesondere eines ersten Drittels, weiter insbesondere innerhalb der Hälfte ausgehend von einem der Enden der ersten Schwächungslinie angeordnet ist oder dass der Kreuzungspunkt die erste Schwächungslinie halbiert.

Es kann auch vorgesehen sein, dass bei einer Ausführungsform mit weiteren zweiten Schwächungslinien, die daraus entstehenden Kreuzungspunkte mit der ersten Schwächungslinie an mehreren Positionen angeordnet sind, dabei mit den Positionen entnommen aus der Gruppe umfassend Kreuzungspunkt innerhalb eines ersten Viertels, Kreuzungspunkt innerhalb eines ersten Drittels, Kreuzungspunkt innerhalb der Hälfte ausgehend von einem der Enden der ersten Schwächungslinie und/oder Kreuzungspunkt auf der Hälfte der ersten Schwächungslinie.

Durch die Positionierung des Kreuzungspunktes kann das Öffnungsverhalten der ersten Schwächungslinie beeinflusst werden. Je geringer die Entfernung des Kreuzungspunktes von einem der Enden der ersten Schwächungslinie ist, desto eher kann ein ungewolltes Öffnen der ersten Schwächungslinie vermieden werden.

Andererseits trägt die Anordnung des, insbesondere eines einzigen, Kreuzungspunkts bei der Hälfte der ersten Schwächungslinie positiv zu der universellen Einsetzbarkeit der Operationsabdeckung bei, da das Entfernen des Schutzmaterialelement gleichartig von beiden Enden begonnen werden kann, und damit auch vorteilhaft sowohl von einem Linkshänder oder einem Rechtshänder unproblematisch vorgenommen werden kann.

Zum anderen wird mit einer Anordnung des Kreuzungspunktes bei der Hälfte der Erstreckung der ersten Schwächungslinie eine einem "Faden/Koordinatenkreuz" ähnliche Anordnung der ersten und zweiten Schwächungslinie erhalten, die der Anwender vorteilhaft als Orientierungshilfe bei der Platzierung der Öffnung bzw. des Folienelements auf dem Patienten einsetzen kann.

Die erste Schwächungslinie und/oder die zweite Schwächungslinie kann gerade, wellenförmig oder gekrümmt oder mit abschnittsweisen Kombinationen davon ausgebildet sein.

Insbesondere weist die erste Schwächungslinie eine alternierende Abfolge von das Folienelement trennenden Bereichen und von das Folienelement verbindenden Stegen auf.

Alternativ oder ergänzend kann die zweite Schwächungslinie eine alternierende Abfolge von das Schutzmaterialelement trennenden Bereichen und von das Schutzmaterialelement verbindenden Stegen aufweisen.

In einer besonders bevorzugten Ausführung sind die erste Schwächungslinie und die zweite Schwächungslinie hinsichtlich dieser alternierenden Abfolge an trennenden Bereichen und verbindenden Stegen identisch ausgebildet.

Bei einer identischen Ausbildung der Abfolge an trennenden Bereichen und verbindenden Stegen der ersten und zweiten Schwächungslinie können die erste und zweite Schwächungslinie in einem einzigen Herstellprozess in die jeweiligen Flachbahnmaterialelemente eingebracht werden.

Bevorzugt weisen der erste Folienelementabschnitt und der zweite Folienelementabschnitt eine gleichgroße Fläche, insbesondere zueinander achsenspiegelsymmetrische Flächen auf.

Gleichgroße Fläche, insbesondere zueinander achsenspiegelsymmetrische Flächen des ersten und zweiten Folienelementabschnittes erleichtern dem Anwender die Positionierung der Öffnung der Operationsabdeckung bzw. des Folienelements am Patienten, da eine universelle Anwendbarkeit unabhängig von der Anordnung des ersten und zweiten Folienelementabschnittes innerhalb der Operationsabdeckung möglich ist.

Das Folienelement kann sich insbesondere über die Öffnung hinaus erstrecken.

Dabei kann das Folienelement vorzugsweise auf der Oberseite des Basistuchelements angeordnet und angebracht sein.

Das Folienelement trägt insbesondere durch dem Material inhärent hydrophobe und/oder durch eingebrachte hydrophobe Eigenschaften zu einer Abdichtung gegenüber während einer Operation auftretenden Flüssigkeiten bei. Mit einem über die Öffnung hinauserstreckenden Folienelement, insbesondere wenn das Folienelement auf der Oberseite des Basistuchelements angebracht ist, werden bei der Operation auftretenden Flüssigkeiten vielmehr von der Öffnung und damit vom Eingriffsfeld weg in entfernt gelegene Bereiche abgeleitet.

Mit dem zumindest innerhalb der Öffnung auf der Unterseite vorhandenen klebenden Bereich, insbesondere der klebenden Schicht des Folienelements kann die Operationsabdeckung an den Patienten fixiert werden.

Ergänzend können vorzugsweise auf der Unterseite des Basistuchelements entlang der Öffnung, vorzugsweise vollumfänglich der Öffnung zusätzlich Klebezonen vorgesehen sein, die eine noch bessere und damit sichere Fixierung der Operationsabdeckung an den Patienten ermöglichen.

In einer besonders bevorzugten Ausführung weist die Operationsabdeckung ausschließlich innerhalb der Öffnung einen klebenden Bereich, insbesondere eine klebende Schicht auf. Die Reduktion des Klebebereichs ausschließlich auf den Bereich der Öffnung trägt zum Komfort des Patienten bei, als dass bei einer möglichst geringen Kleberfläche auch weniger Belastung auf die Haut des Patienten ausgeübt wird und die Fläche des der Operation nachgeschalteten zumeist nicht immer schmerzfreien Ablösevorgangs der Operationsabdeckung vom Patienten minimiert ist. Zudem spart diese bevorzugte Ausführungsform Material und damit Kosten ein.

Alternativ sind auch bevorzugte Ausführungsformen denkbar, bei der das Folienelement auf der Unterseite des Basistuchelements angebracht ist. Insbesondere in der Ausführungsform, dass das Folienelement auf der Unterseite in über die Öffnung hinausreichenden Abschnitten mit einem klebenden Bereich, insbesondere einer klebenden Schicht ausgestattet ist, trägt dies vorteilhaft zu einem verbesserten Festlegen und Fixieren der Operationsabdeckung am Patienten bei.

Insbesondere bevorzugt erstreckt sich das Schutzmaterialelement über den auf der Unterseite vorhandenen klebenden Bereich, insbesondere die klebende Schicht hinaus.

Mit dem Überfangen des Folienelements bzw. dessen klebenden Bereichs, insbesondere klebenden Schicht mit einem Schutzmaterialelement mit einer vergleichsweise größeren Erstreckung können dem Anwender vorteilhaft Anfasshilfen bereitgestellt werden.

Das Schutzmaterialelement kann Papier oder polymeres Material entnommen aus der Gruppe Polyethylen, Polypropylen, Polyurethan oder Mischungen umfassen oder daraus gebildet sein. Insbesondere kann das Schutzmaterialelement mit einer Beschichtung für Releaseverhalten, insbesondere mit Siloxanen ausgestattet sein.

Vorzugsweise weist das Schutzmaterialelement ein Flächengewicht von mindestens 20 g/m², insbesondere mindestens 30 g/m², weiter insbesondere mindestens 40 g/m², weiter insbesondere mindestens 50 g/m², weiter insbesondere von höchstens 100 g/m², weiter insbesondere höchstens 90 g/m², weiter insbesondere höchstens 80 g/m² auf.

Das Schutzmaterialelement weist vorzugsweise eine Dicke von mindestens 25 µm, insbesondere mindestens 30 µm, weiter insbesondere mindestens 35 µm, weiter insbesondere mindestens 40 µm, weiter insbesondere höchstens 100 µm, weiter insbesondere höchstens 90 µm, weiter insbesondere höchstens 80 µm auf.

Insbesondere bevorzugt weist das Schutzmaterialelement im Vergleich zum Folienelement ein größeres Flächengewicht und/oder eine größere Dicke auf. Damit kann vorteilhaft eine Stabilisierung von dünneren und flexiblen Folienelementen geschaffen werden. Eine mit Flächengewicht und/oder Dicke einhergehende Steifigkeit des Schutzmaterialelements ist für die Anwendung des Schutzmaterialelements als Applikationshilfe besonders positiv zuträglich.

Das Folienelement besteht vorzugsweise aus einem polymeren Material, insbesondere aus polymerem Material entnommen aus der Gruppe Polyethylen, Polypropylen, Polyurethan oder Mischungen davon.

Das Folienelement weist vorzugsweise ein Flächengewicht von mindestens 10 g/m², insbesondere mindestens 15 g/m², weiter insbesondere mindestens 20 g/m², weiter insbesondere mindestens 25 g/m², weiter insbesondere von höchstens 50 g/m², weiter insbesondere höchstens 45 g/m², weiter insbesondere höchstens 40 g/m², weiter insbesondere höchstens 35 g/m² auf.

Das Folienelement weist vorzugsweise eine Dicke von mindestens 5 µm, insbesondere mindestens 10 µm, weiter insbesondere mindestens 15 µm, weiter insbesondere mindestens 20 µm, weiter insbesondere höchstens 50 µm, weiter insbesondere höchstens 40 µm, weiter insbesondere höchstens 30 µm auf.

Die Dickenmessung wird mittels eines mechanischen Dickenmessgeräts unter Anwendung eines spezifischen Messdrucks von 2 g/cm² auf einer Tasterfläche von 25 cm² durchgeführt.

Vorzugsweise ist das Folienelement transparent.

Damit ist bereits beim Anbringen des Folienelements auf den Patienten für den Anwender eine bessere Orientierung möglich. Zudem verschafft die Transparenz eine Einsehbarkeit in die am Eingriffsbereich direkt benachbarte Umgebung.

Die Öffnung weist vorzugsweise eine polygonale oder runde, insbesondere eine kreisrunde oder ovale Form auf.

Polygonale oder runde Öffnungen belassen dem Anwender eine universelle Anwendbarkeit der Operationsabdeckung bzw. eine individuelle Positionierung der Öffnung auf dem Patienten. Zudem sind polygonale oder runde Öffnungen im Herstellungsprozess leichter in das Basistuchelement einzubringen als eine Öffnung mit einer eher komplizierten Kontur.

Die Öffnung weist vorzugsweise eine Fläche von mindestens 15 cm², insbesondere mindestens 20 cm², weiter insbesondere mindestens 25 cm², weiter insbesondere mindestens 30 cm², und weiter insbesondere höchstens 100 cm², weiter insbesondere höchstens 90 cm², weiter insbesondere höchstens 80 cm², weiter insbesondere höchstens 70 cm², weiter insbesondere höchstens 60 cm² auf.

Der klebende Bereich, insbesondere die klebende Schicht auf dem Folienelement ist vorzugsweise eine Haftkleberbeschichtung, insbesondere ein Polyacrylatkleber, insbesondere ein UV-vernetzter Polyacrylatkleber.

Der klebende Bereich, insbesondere die klebende Schicht auf dem Folienelement weist ein Flächengewicht von mindestens 10 g/m², insbesondere mindestens 15 g/m², weiter insbesondere mindestens 20 g/m², weiter insbesondere mindestens 25 g/m², weiter insbesondere von höchstens 60 g/m², weiter insbesondere höchstens 55 g/m², weiter insbesondere höchstens 50 g/m², weiter insbesondere höchstens 45 g/m², weiter insbesondere höchstens 40 g/m² auf.

Vorzugsweise können die Flächengewichte des Folienelements und des klebenden Bereichs, insbesondere der klebenden Schicht weitgehend übereinstimmen, bevorzugt in einer Abweichung von höchstens 10%.

Bei der Operationsabdeckung handelt es sich vorzugsweise um ein Augen-Operationstuch, ein Nasen-Operationstuch, ein Ohr-Operationstuch, ein Fersen-Operationstuch, ein Handgelenk-Operationstuch oder ein Knöchel-Operationstuch.

Bei der Operationsabdeckung handelt es sich vorzugsweise um eine wegwerfbare Operationsabdeckung.

Das bedeutet, dass die Operationsabdeckung zum insbesondere einmaligen Gebrauch bestimmt ist. Das heißt eine wegwerfbare Operationsabdeckung ist nicht dafür geeignet und vorgesehen nach Gebrauch gereinigt oder gewaschen und anschließend wiederverwendet zu werden.

Für das Basistuchelement werden bevorzugt Vliesmaterialien und/ oder Vlies-Folien-Verbundmaterialien eingesetzt. Insbesondere bei wegwerfbaren Operationsabdeckungen werden für das Basistuchelement Vliesmaterialien und/oder Vlies-Folien-Verbundmaterialien eingesetzt.

Als Vliesmaterialien können vorzugsweise ein ein- oder mehrlagiger Vliesstoff, insbesondere ein Laminat aus einer oder mehreren Spinnvlies- und/oder Meltblownvlieslagen und/oder als Vlies-Folien-Verbundmaterialien, insbesondere Vlies-Folien-Verbundmaterialien mit einem hydrophilem Vliesstoff mit ein oder mehreren Lagen aus Spinnvlies und/oder Meltblown eingesetzt werden. Spinnvlies- und/oder Meltblownvlieslagen umfassen vorzugsweise polymere Materialien, insbesondere ausgewählt aus der Gruppe Polyethylen, Polypropylen oder Mischungen, insbesondere ein hydrophilisiertes Polypropylen.

Das Basistuchelement weist vorzugsweise ein Flächengewicht von mindestens 20 g/m², insbesondere mindestens 30 g/m², weiter insbesondere mindestens 40 g/m², weiter insbesondere von höchstens 120 g/m², weiter insbesondere höchstens 100 g/m², weiter insbesondere höchstens 80 g/m², weiter insbesondere höchstens 60 g/m² auf.

Weitere Merkmale, Vorteile und Einzelheiten der Erfindung ergeben sich aus den beigefügten Patentansprüchen und aus der zeichnerischen Darstellung und der nachfolgenden Beschreibung bevorzugter Ausführungsformen der Erfindung.

Die Erfindung soll im Folgenden anhand von Figuren näher erläutert werden, dabei zeigen
- Figur 1:: schematisch eine erfindungsmäße Operationsabdeckung in einer Draufsicht mit sich kreuzender erster und zweiter Schwächungslinie,
- Figuren 2a und 2b:: schematisch Ausführungsformen einer Schnittansicht I - I von Figur 1,
- Figur 3:: schematisch eine Schnittansicht einer weiteren erfindungsgemäßen Operationsabdeckung,
- Figur 4a:: schematisch eine erfindungsmäße Operationsabdeckung mit einem gefalteten Schutzmaterialelement und
- Figur 4b:: schematisch eine Schnittansicht II - II von Figur 4a.

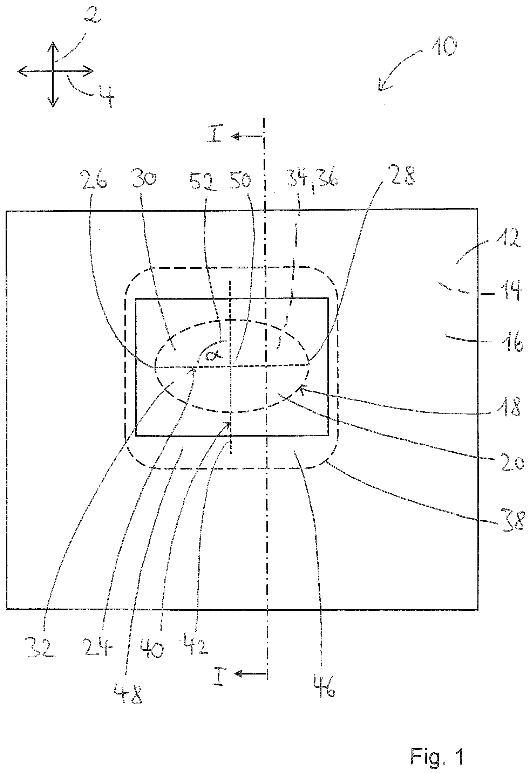

Figur 1 zeigt schematisch eine Draufsicht einer bevorzugten Operationsabdeckung 10 mit einer Oberseite 12 und einer Unterseite 14 und mit einem Basistuchelement 16 mit einer Öffnung 18. Die Öffnung 18 mit einer vorzugsweisen runden, insbesondere einer ovalen Form, insbesondere mit einer bevorzugten Fläche von 20 - 60 cm², ist von einem Folienelement 20 überfangen. Das Folienelement weist eine erste Schwächungslinie 24 auf, entlang derer das Folienelement 20 in einen ersten Folienelementabschnitt 30 und in einen zweiten Folienelementabschnitt 32 trennbar ist. In einer bevorzugten Ausführungsform weisen der erste und zweite Folienelementabschnitt 30, 32 zwei gleichgroße, insbesondere zueinander achsenspiegelsymmetrisch gleichgroße Flächen auf. Auf der Unterseite 14 des Folienelements 20 ist ein klebender Bereich 34, insbesondere eine klebende Schicht 36 vorhanden, wobei dieser klebende Bereich 34 bzw. die klebende Schicht 36 mit einem Schutzmaterialelement 38 abgedeckt ist. Das Schutzmaterialelement 38 weist eine zweite Schwächungslinie 40 auf. Das Schutzmaterialelement 38 und das Folienelement 20 sind derart zueinander angeordnet, dass die zweite Schwächungslinie 40 die erste Schwächungslinie 24 kreuzt. In der Operationsabdeckung 10 mit einer Längsrichtung 2 und einer Querrichtung 4 verlaufen vorzugsweise die erste Schwächungslinie 24 zumindest bereichsweise, oder bevorzugt ausschließlich in Längsrichtung 2 oder Querrichtung 4 und die zweite Schwächungslinie 40 verläuft zumindest bereichsweise, oder bevorzugt ausschließlich in der jeweils anderen Querrichtung 4 oder Längsrichtung 2. Die erste und zweite Schwächungslinie können unterschiedlich ausgebildet, oder alternativ und insbesondere bevorzugt identisch ausgebildet sein. Die zweite Schwächungslinie 40 weist insbesondere einen Verlauf 42 auf, welcher an der ersten Schwächungslinie 24 achsensymmetrisch gespiegelt ist. Das bedeutet, dass die zweite Schwächungslinie 40 auf die erste Schwächungslinie 24 ausgehend von einem der Enden 26, 28 der ersten Schwächungslinie 24 in einem Kreuzungspunkt 50 in einem Winkel α 52 auftrifft und die zweite Schwächungslinie 40 ausgehend von diesem Kreuzungspunkt 50 achsensymmetrisch an der ersten Schwächungslinie 24 in ihrem Verlauf 42 fortgeführt wird. Es sind auch Anordnungen der Schwächungslinien zueinander denkbar, in denen die zweite Schwächungslinie die erste Schwächungslinie in einem Kreuzungspunkt 50 kreuzt und die zweite Schwächungslinie diesen Verlauf punktsymmetrisch an diesem Kreuzungspunkt über die erste Schwächungslinie hinweg beibehält. Der Winkel α weist dabei vorzugsweise 45 - 90° auf. In einer besonders bevorzugten Ausführung, wie dargestellt, kreuzen sich die beiden Schwächungslinien in einem Winkel α von 90°, sie sind also in einem rechten Winkel zueinander angeordnet. Das Schutzmaterialelement 38 weist vorzugsweise einen um die zweite Schwächungslinie 40 faltbaren, oder insbesondere trennbaren ersten Schutzmaterialelementabschnitt 46 und einen zweiten Schutzmaterialelementabschnitt 48 auf. Der Kreuzungspunkt 50 der zweiten Schwächungslinie 40 mit der ersten Schwächungslinie 24 ist zwischen den Enden 26, 28 der ersten Schwächungslinie 24, besonders bevorzugt bei der Hälfte der Erstreckung der ersten Schwächungslinie 24 angeordnet. Es ist aber auch ein Kreuzungspunkt innerhalb eines ersten Viertels, insbesondere innerhalb eines ersten Drittels, insbesondere innerhalb der Hälfte ausgehend von einem der Enden der ersten Schwächungslinie denkbar.

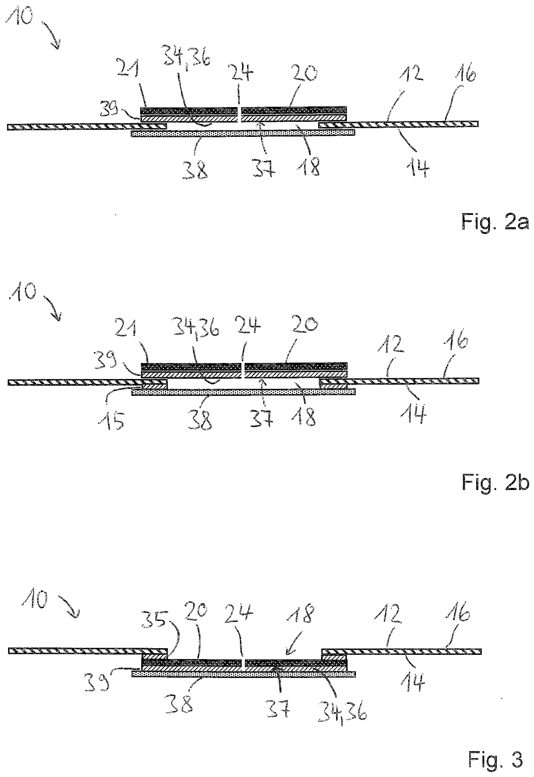

Die Figuren 2a und 2b beziehen sich auf die Schnittansicht I - I von Figur 1. Es sind mögliche Ausführungsformen der Abfolge einzelner Komponenten dargestellt: Das jeweils auf der Oberseite 12 des Basistuchelements 16 angeordnete und dabei die Öffnung 18 überfangende Folienelement 20 ist auf der Unterseite 14 mit einem klebenden Bereich 34, insbesondere mit einer vollflächigen klebenden Schicht 36 belegt. Damit weist das Folienelement zumindest innerhalb der Öffnung 18 einen klebenden Bereich 34, insbesondere eine klebende Schicht 36 auf. Mit dem die Öffnung 18 überfangenen Bereich 37 der klebenden Schicht 36 kann die Operationsabdeckung im Anwendungszustand an den Patienten fixiert werden.

In den über die Öffnung 18 hinauserstreckenden Bereichen 21 des Folienelements 20 sind vorzugsweise ebenso klebende Bereiche 39 vorgesehen, mit denen das Folienelement 20 an das Basistuchelement 16 festgelegt ist. Im Vergleich zu Figur 2a ist in Figur 2b das Basistuchelement 16 auf der Unterseite 14 und dabei in an die Öffnung 18 angrenzenden Bereichen mit einem weiteren klebenden Bereich 15 ausgestattet. Dieser klebende Bereich 15 dient der weiteren Fixierung der Operationsabdeckung an den Patienten. Den klebenden Bereichen 15 und 37 in Figur 2b oder dem klebenden Bereich 37 in Figur 2a ist jeweils ein Schutzmaterialelement 38 zugeordnet. Der in den Figuren 2a und 2b skizzierte Abstand zwischen den klebenden Bereichen 37 und dem Schutzmaterialelement 38 ist der lediglich schematischen Darstellung geschuldet, tatsächlich aber nicht vorhanden. Die Positionierung der ersten Schwächungslinie 24 ist in diesen Darstellungen schematisch angedeutet.

In Figur 3 ist eine weitere alternative Ausführungsform der Abfolge einzelner Komponenten schematisch dargestellt: Die in das Basistuchelement 16 eingebrachte Öffnung 18 ist auf der Unterseite 14 mit einem Folienelement 20 überfangen. Das Folienelement 20 ist dabei mittels klebenden Bereichen 35 an das die Öffnung 18 abgrenzende Basistuchelement fixiert. Der klebende Bereich 34, insbesondere die klebende Schicht 36 des Folienmaterials 20 ist mit einem Schutzmaterialelement 38 abgedeckt.

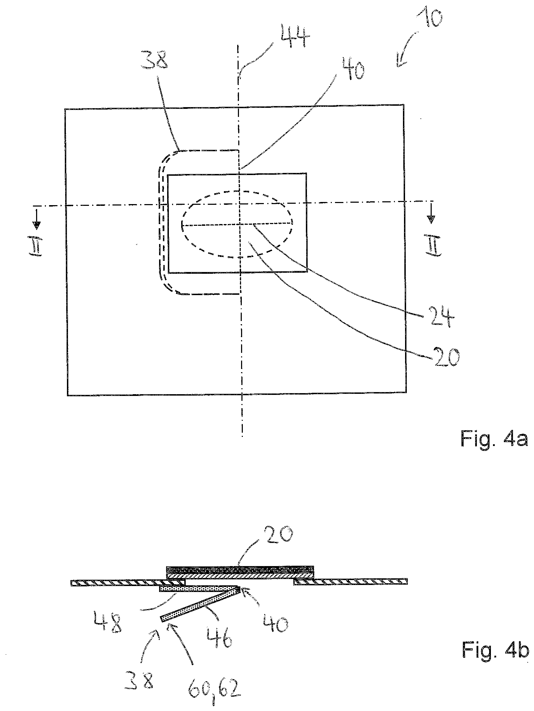

Das Schutzmaterialelement 38 kann vorteilhaft an einer durch die zweite Schwächungslinie 40 beschriebenen Achse 44 gefaltet werden, wie schematisch in Figur 4a in einer Draufsicht und schematisch in Figur 4b als Schnittansicht II - II von Figur 4a dargestellt ist. Dabei teilt die zweite Schwächungslinie 40 das Schutzmaterialelement 38 in einen ersten Schutzmaterialelementabschnitt 46 und in einen zweiten Schutzmaterialelementabschnitt 48. In einer solchen Konfiguration kann der erste Schutzmaterialabschnitt 46 als Applikationshilfe 60, insbesondere als ein Anfassmittel 62 genutzt werden. Insbesondere vorteilhaft weist das Schutzmaterialelement ein zum Folienelement vergleichsweise größeres Flächengewicht und damit auch eine größere Dicke auf. Eine damit einhergehende Steifigkeit des Schutzmaterialelements ist für die Anwendung des Schutzmaterialelements als Applikationshilfe besonders positiv zuträglich.

In einer bevorzugten Ausführung eines transparenten Folienelements 20 wird durch das Umfalten des ersten Schutzmaterialelementabschnitts 46 eines vergleichsweise blickdichteren Schutzmaterialelements 38 die Transparenz des Folienelements freigelegt und gewährt dem Anwender damit eine Einsehbarkeit auf den vorgesehenen Eingriffsbereich. Dies ist für die bereichsweise Positionierung, Andrapierung und dann Fixierung der Operationsabdeckung an den Patienten hilfreich.

## Patentansprüche

1. Operationsabdeckung (10) zur zumindest bereichsweisen Abdeckung eines menschlichen oder tierischen Körpers, mit einer Oberseite (12) und einer Unterseite (14), mit einem Basistuchelement (16) und mit zumindest einer innerhalb des Basistuchelements (16) angeordneten Öffnung (18), wobei diese Öffnung (18) von einem Folienelement (20) überfangen ist und wobei dieses Folienelement (20) eine erste Schwächungslinie (24) aufweist und wobei die erste Schwächungslinie (24) im Bereich der Öffnung (18) angeordnet ist und wobei das Folienelement (20) auf der Unterseite (14) zumindest innerhalb der Öffnung (18) einen klebenden Bereich (34), insbesondere eine klebende Schicht (36) aufweist und wobei auf dem klebenden Bereich (34), insbesondere der klebenden Schicht (36) ein ablösbares Schutzmaterialelement (38) angeordnet ist, wobei das Schutzmaterialelement (38) eine zweite Schwächungslinie (40) aufweist, **dadurch gekennzeichnet, dass** das Schutzmaterialelement (38) derart auf dem Folienelement (20) angeordnet ist, dass die zweite Schwächungslinie (40) die erste Schwächungslinie (24) kreuzt.

2. Operationsabdeckung (10) nach Anspruch 1, **dadurch gekennzeichnet, dass** das Folienelement (20) einen ersten Folienelementabschnitt (30) und einen zweiten Folienelementabschnitt (32) aufweist, wobei der erste Folienelementabschnitt (30) entlang der ersten Schwächungslinie (24) von dem zweiten Folienelementabschnitt (32) trennbar ist.

3. Operationsabdeckung (10) nach Anspruch 1 oder 2, **dadurch gekennzeichnet, dass** das Schutzmaterialelement (38) einen ersten Schutzmaterialelementabschnitt (46) und einen zweiten Schutzmaterialelementabschnitt (48) aufweist, wobei der erste Schutzmaterialelementabschnitt (46) entlang der zweiten Schwächungslinie (40) von dem zweiten Schutzmaterialelementabschnitt (48) trennbar ist.

4. Operationsabdeckung (10) nach einem der vorgenannten Ansprüche, **dadurch gekennzeichnet, dass** die Operationsabdeckung (10) eine Längsrichtung (2) und eine Querrichtung (4) aufweist und die erste Schwächungslinie (24) zumindest bereichsweise, insbesondere ausschließlich in Querrichtung (4) oder Längsrichtung (2) verläuft und die zweite Schwächungslinie (40) zumindest bereichsweise, insbesondere ausschließlich in der jeweils anderen Längsrichtung (2) oder Querrichtung (4) verläuft.

5. Operationsabdeckung (10) nach einem der vorgenannten Ansprüche, **dadurch gekennzeichnet, dass** die zweite Schwächungslinie (40) einen an der ersten Schwächungslinie (24) achsensymmetrisch gespiegelten Verlauf (42) aufweist oder dass die zweite Schwächungslinie (40) einen in einem Kreuzungspunkt (50) mit der ersten Schwächungslinie (24) punktsymmetrisch gespiegelten Verlauf (42) beibehält.

6. Operationsabdeckung (10) nach einem der vorgenannten Ansprüche, **dadurch gekennzeichnet, dass** die zweite Schwächungslinie (40) die erste Schwächungslinie (24) ausgehend von einem der Enden (26, 28) der ersten Schwächungslinie (24) in einem Winkel α (52) von 45° - 90 ° kreuzt, insbesondere dass die zweite Schwächungslinie (40) die erste Schwächungslinie (24) in einem Winkel α von 90° kreuzt.

7. Operationsabdeckung (10) nach einem der vorgenannten Ansprüche, **dadurch gekennzeichnet, dass** die zweite Schwächungslinie (40) die erste Schwächungslinie (24) derart in einem Kreuzungspunkt (50) kreuzt, dass der Kreuzungspunkt (50) die erste Schwächungslinie (24) halbiert.

8. Operationsabdeckung (10) nach einem der vorgenannten Ansprüche, **dadurch gekennzeichnet, dass** die erste Schwächungslinie (24) eine alternierende Abfolge von das Folienelement (20) trennenden Bereichen und von das Folienelement (20) verbindenden Stegen aufweist und dass die zweite Schwächungslinie (40) eine alternierende Abfolge von das Schutzmaterialelement (38) trennenden Bereichen und von das Schutzmaterialelement (40) verbindenden Stegen aufweist, und insbesondere dass die erste Schwächungslinie (24) und die zweite Schwächungslinie (40) hinsichtlich dieser Abfolge an trennenden Bereichen und verbindenden Stegen identisch ausgebildet sind.

9. Operationsabdeckung (10) nach Anspruch 2, **dadurch gekennzeichnet, dass** der erste Folienelementabschnitt (30) und der zweite Folienelementabschnitt (32) eine gleichgroße Fläche, insbesondere zueinander achsenspiegelsymmetrische Flächen aufweisen.

10. Operationsabdeckung (10) nach einem der vorgenannten Ansprüche, **dadurch gekennzeichnet, dass** das Schutzmaterialelement (38) sich über den auf der Unterseite (14) vorhandenen klebenden Bereich (34), insbesondere der klebenden Schicht (36) hinauserstreckt.

11. Operationsabdeckung (10) nach einem der vorgenannten Ansprüche, **dadurch gekennzeichnet, dass** das Schutzmaterialelement (38) Papier oder polymeres Material entnommen aus der Gruppe Polyethylen, Polypropylen, Polyurethan oder Mischungen davon umfasst oder daraus gebildet ist.

12. Operationsabdeckung (10) nach einem der vorgenannten Ansprüche, **dadurch gekennzeichnet, dass** das Schutzmaterialelement (38) ein Flächengewicht von mindestens 20 g/m², insbesondere mindestens 30 g/m², weiter insbesondere mindestens 40 g/m², weiter insbesondere mindestens 50 g/m², weiter insbesondere von höchstens 100 g/m², weiter insbesondere höchstens 90 g/m², weiter insbesondere höchstens 80 g/m² aufweist.

13. Operationsabdeckung (10) nach einem der vorgenannten Ansprüche, **dadurch gekennzeichnet, dass** das Folienelement (20) ein Flächengewicht von mindestens 10 g/m², insbesondere mindestens 15 g/m², weiter insbesondere mindestens 20 g/m², weiter insbesondere mindestens 25 g/m², weiter insbesondere von höchstens 50 g/m², weiter insbesondere höchstens 45 g/m², weiter insbesondere höchstens 40 g/m², weiter insbesondere höchstens 35 g/m² aufweist.

14. Operationsabdeckung (10) nach einem der vorgenannten Ansprüche, **dadurch gekennzeichnet, dass** das Folienelement (20) transparent ist.

15. Operationsabdeckung (10) nach einem der vorgenannten Ansprüche, **dadurch gekennzeichnet, dass** die Öffnung (18) eine polygonale oder runde, insbesondere eine kreisrunde oder ovale Form aufweist.

16. Operationsabdeckung (10) nach einem der vorgenannten Ansprüche, **dadurch gekennzeichnet, dass** die Öffnung (18) eine Fläche von mindestens 15 cm², insbesondere mindestens 20 cm², weiter insbesondere mindestens 25 cm², weiter insbesondere mindestens 30 cm², und weiter insbesondere höchstens 100 cm², weiter insbesondere höchstens 90 cm², weiter insbesondere höchstens 80 cm², weiter insbesondere höchstens 70 cm², weiter insbesondere höchstens 60 cm² aufweist

17. Operationsabdeckung (10) nach einem der vorgenannten Ansprüche, **dadurch gekennzeichnet, dass** die Operationsabdeckung (10) ein Augen-Operationstuch, ein Nasen-Operationstuch, ein Ohr-Operationstuch, ein Fersen-Operationstuch, ein Handgelenk-Operationstuch oder ein Knöchel-Operationstuch ist.

18. Operationsabdeckung (10) nach einem der vorgenannten Ansprüche, **dadurch gekennzeichnet, dass** das Schutzmaterialelement (38) einen ersten Schutzmaterialelementabschnitt (46) und einen zweiten Schutzmaterialelementabschnitt (48) aufweist, wobei der erste Schutzmaterialelementabschnitt (46) entlang der zweiten Schwächungslinie (40) in Richtung auf den zweiten Schutzmaterialelementabschnitt (48) faltbar ist und der erste Schutzmaterialelementabschnitt (46) in dieser Konfiguration als eine Applikationshilfe (60), insbesondere als ein Anfassmittel (62) nutzbar ist.

## Claims

1. Surgical drape (10) for covering a human or animal body at least in some regions, having a top side (12), a bottom side (14), a base cloth element (16), and at least one opening (18) arranged within the base cloth element (16), wherein said opening (18) is covered by a film element (20), and wherein said film element (20) has a first weakening line (24), and wherein the first weakening line (24) is arranged in the region of the opening (18), and wherein the film element (20) has an adhesive region (34), in particular an adhesive layer (36), on the bottom side (14) at least within the opening (18), and wherein a releasable protective material element (38) is arranged on the adhesive region (34), in particular the adhesive layer (36), wherein the protective material element (38) has a second weakening line (40), **characterized in that** the protective material element (38) is arranged on the film element (20) in such a way that the second weakening line (40) crosses the first weakening line (24) .

2. Surgical drape (10) according to Claim 1, **characterized in that** the film element (20) has a first film element portion (30) and a second film element portion (32), wherein the first film element portion (30) is separable from the second film element portion (32) along the first weakening line (24) .

3. Surgical drape (10) according to Claim 1 or 2, **characterized in that** the protective material element (38) has a first protective material element portion (46) and a second protective material element portion (48), wherein the first protective material element portion (46) is separable from the second protective material element portion (48) along the second weakening line (40).

4. Surgical drape (10) according to one of the preceding claims, **characterized in that** the surgical drape (10) has a longitudinal direction (2) and a transverse direction (4), and the first weakening line (24) runs at least in some regions, in particular exclusively, in the transverse direction (4) or longitudinal direction (2), and the second weakening line (40) runs at least in some regions, in particular exclusively, in the respective other longitudinal direction (2) or transverse direction (4) .

5. Surgical drape (10) according to one of the preceding claims, **characterized in that** the second weakening line (40) has a course (42) mirrored with axial symmetry on the first weakening line (24), or in that the second weakening line (40) maintains a course (42) mirrored with point symmetry at an intersection (50) with the first weakening line (24) .

6. Surgical drape (10) according to one of the preceding claims, **characterized in that** the second weakening line (40) crosses the first weakening line (24) proceeding from one of the ends (26, 28) of the first weakening line (24) at an angle α (52) of 45°-90°, in particular **in that** the second weakening line (40) crosses the first weakening line (24) at an angle α of 90°.

7. Surgical drape (10) according to one of the preceding claims, **characterized in that** the second weakening line (40) crosses the first weakening line (24) at an intersection (50) in such a way that the intersection (50) halves the first weakening line (24) .

8. Surgical drape (10) according to one of the preceding claims, **characterized in that** the first weakening line (24) has an alternating sequence of regions separating the film element (20) and of webs connecting the film element (20), and **in that** the second weakening line (40) has an alternating sequence of regions separating the protective material element (38) and of webs connecting the protective material element (40), and in particular in that the first weakening line (24) and the second weakening line (40) are configured identically in terms of this sequence of separating regions and connecting webs.

9. Surgical drape (10) according to Claim 2, **characterized in that** the first film element portion (30) and the second film element portion (32) have a surface area of equal size, in particular surfaces that are mirrored with axial symmetry to each other.

10. Surgical drape (10) according to one of the preceding claims, **characterized in that** the protective material element (38) extends over the adhesive region (34), in particular the adhesive layer (36), present on the bottom side (14).

11. Surgical drape (10) according to one of the preceding claims, **characterized in that** the protective material element (38) comprises or is formed from paper or polymer material taken from the group of polyethylene, polypropylene, polyurethane or mixtures of these.

12. Surgical drape (10) according to one of the preceding claims, **characterized in that** the protective material element (38) has a basis weight of at least 20 g/m², particularly at least 30 g/m², more particularly at least 40 g/m², more particularly at least 50 g/m², more particularly at most 100 g/m², more particularly at most 90 g/m², more particularly at most 80 g/m².

13. Surgical drape (10) according to one of the preceding claims, **characterized in that** the film element (20) has a basis weight of at least 10 g/m², particularly at least 15 g/m², more particularly at least 20 g/m², more particularly at least 25 g/m², more particularly at most 50 g/m², more particularly at most 45 g/m², more particularly at most 40 g/m², more particularly at most 35 g/m².

14. Surgical drape (10) according to one of the preceding claims, **characterized in that** the film element (20) is transparent.

15. Surgical drape (10) according to one of the preceding claims, **characterized in that** the opening (18) has a polygonal or round shape, in particular a circular or oval shape.

16. Surgical drape (10) according to one of the preceding claims, **characterized in that** the opening (18) has an area of at least 15 cm², particularly at least 20 cm², more particularly at least 25 cm², more particularly at least 30 cm², and more particularly at most 100 cm², more particularly at most 90 cm², more particularly at most 80 cm², more particularly at most 70 cm², more particularly at most 60 cm².

17. Surgical drape (10) according to one of the preceding claims, **characterized in that** the surgical drape (10) is a drape for eye surgery, a drape for nasal surgery, a drape for ear surgery, a drape for heel surgery, a drape for wrist surgery or a drape for ankle surgery.

18. Surgical drape (10) according to one of the preceding claims, **characterized in that** the protective material element (38) has a first protective material element portion (46) and a second protective material element portion (48), wherein the first protective material element portion (46) is foldable along the second weakening line (40) in the direction of the second protective material element portion (48), and the first protective material element portion (46) in this configuration is usable as an application aid (60), in particular as a tactile means (62).

## Revendications

1. Couverture opératoire (10) pour le recouvrement au moins en zones d'un corps humain ou animal, comprenant un côté supérieur (12) et un côté inférieur (14), comprenant un élément champ de base (16) et comprenant au moins une ouverture (18) agencée à l'intérieur de l'élément champ de base (16), cette ouverture (18) étant doublée par un élément film (20), et cet élément film (20) comprenant une première ligne d'affaiblissement (24), et la première ligne d'affaiblissement (24) étant agencée dans la zone de l'ouverture (18), et l'élément film (20) comprenant une zone adhésive (34), en particulier une couche adhésive (36), sur le côté inférieur (14), au moins à l'intérieur de l'ouverture (18), et un élément matériau de protection amovible (38) étant agencé sur la zone adhésive (34), en particulier la couche adhésive (36), l'élément matériau de protection (38) comprenant une deuxième ligne d'affaiblissement (40), **caractérisée en ce que** l'élément matériau de protection (38) est agencé sur l'élément film (20) de telle sorte que la deuxième ligne d'affaiblissement (40) croise la première ligne d'affaiblissement (24).

2. Couverture opératoire (10) selon la revendication 1, **caractérisée en ce que** l'élément film (20) comprend une première section d'élément film (30) et une deuxième section d'élément film (32), la première section d'élément film (30) étant séparable de la deuxième section d'élément film (32) le long de la première ligne d'affaiblissement (24).

3. Couverture opératoire (10) selon la revendication 1 ou 2, **caractérisée en ce que** l'élément matériau de protection (38) comprend une première section d'élément matériau de protection (46) et une deuxième section d'élément matériau de protection (48), la première section d'élément matériau de protection (46) étant séparable de la deuxième section d'élément matériau de protection (48) le long de la deuxième ligne d'affaiblissement (40).

4. Couverture opératoire (10) selon l'une quelconque des revendications précédentes, **caractérisée en ce que** la couverture opératoire (10) présente une direction longitudinale (2) et une direction transversale (4), et la première ligne d'affaiblissement (24) s'étend au moins en zones, en particulier exclusivement dans la direction transversale (4) ou direction longitudinale (2), et la deuxième ligne d'affaiblissement (40) s'étend au moins en zones, en particulier exclusivement dans l'autre direction longitudinale (2) ou direction transversale (4) respective.

5. Couverture opératoire (10) selon l'une quelconque des revendications précédentes, **caractérisée en ce que** la deuxième ligne d'affaiblissement (40) présente un parcours (42) reflété par symétrie axiale par rapport à la première ligne d'affaiblissement (24), ou **en ce que** la deuxième ligne d'affaiblissement (40) maintient un parcours (42) reflété par symétrie ponctuelle par rapport à un point de croisement (50) avec la première ligne d'affaiblissement (24).

6. Couverture opératoire (10) selon l'une quelconque des revendications précédentes, **caractérisée en ce que** la deuxième ligne d'affaiblissement (40) croise la première ligne d'affaiblissement (24) en partant d'une des extrémités (26, 28) de la première ligne d'affaiblissement (24) en un angle α (52) de 45° à 90°, en particulier **en ce que** la deuxième ligne d'affaiblissement (40) croise la première ligne d'affaiblissement (24) en un angle α de 90°.

7. Couverture opératoire (10) selon l'une quelconque des revendications précédentes, **caractérisée en ce que** la deuxième ligne d'affaiblissement (40) croise la première ligne d'affaiblissement (24) en un point de croisement (50) de telle sorte que le point de croisement (50) divise en deux la première ligne d'affaiblissement (24).

8. Couverture opératoire (10) selon l'une quelconque des revendications précédentes, **caractérisée en ce que** la première ligne d'affaiblissement (24) présente une succession alternée de zones de séparation de l'élément film (20) et de traverses de liaison de l'élément film (20), et **en ce que** la deuxième ligne d'affaiblissement (40) présente une succession alternée de zones de séparation de l'élément matériau de protection (38) et de traverses de liaison de l'élément matériau de protection (40), et en particulier **en ce que** la première ligne d'affaiblissement (24) et la deuxième ligne d'affaiblissement (40) sont configurées de manière identique au regard de cette succession de zones de séparation et de zones de liaison.

9. Couverture opératoire (10) selon la revendication 2, **caractérisée en ce que** la première section d'élément film (30) et la deuxième section d'élément film (32) présentent une surface de même taille, en particulier des surfaces symétriques axialement l'une de l'autre.

10. Couverture opératoire (10) selon l'une quelconque des revendications précédentes, **caractérisée en ce que** l'élément matériau de protection (38) s'étend au-delà de la zone adhésive (34), en particulier de la couche adhésive (36), présente sur le côté inférieur (14).

11. Couverture opératoire (10) selon l'une quelconque des revendications précédentes, **caractérisée en ce que** l'élément matériau de protection (38) comprend du papier ou un matériau polymère extrait du groupe constitué par le polyéthylène, le polypropylène, le polyuréthane ou des mélanges de ceux-ci, ou en est formé.

12. Couverture opératoire (10) selon l'une quelconque des revendications précédentes, **caractérisée en ce que** l'élément matériau de protection (38) présente un poids superficiel d'au moins 20 g/m², en particulier d'au moins 30 g/m², plus particulièrement d'au moins 40 g/m², plus particulièrement d'au moins 50 g/m², plus particulièrement d'au plus 100 g/m², plus particulièrement d'au plus 90 g/m², plus particulièrement d'au plus 80 g/m².

13. Couverture opératoire (10) selon l'une quelconque des revendications précédentes, **caractérisée en ce que** l'élément film (20) présente un poids superficiel d'au moins 10 g/m², en particulier d'au moins 15 g/m², plus particulièrement d'au moins 20 g/m², plus particulièrement d'au moins 25 g/m², plus particulièrement d'au plus 50 g/m², plus particulièrement d'au plus 45 g/m², plus particulièrement d'au plus 40 g/m², plus particulièrement d'au plus 35 g/m².

14. Couverture opératoire (10) selon l'une quelconque des revendications précédentes, **caractérisée en ce que** l'élément film (20) est transparent.

15. Couverture opératoire (10) selon l'une quelconque des revendications précédentes, **caractérisée en ce que** l'ouverture (18) présente une forme polygonale ou ronde, en particulier une forme circulaire ou ovale.

16. Couverture opératoire (10) selon l'une quelconque des revendications précédentes, **caractérisée en ce que** l'ouverture (18) présente une surface d'au moins 15 cm², en particulier d'au moins 20 cm², plus particulièrement d'au moins 25 cm², plus particulièrement d'au moins 30 cm², et plus particulièrement d'au plus 100 cm², plus particulièrement d'au plus 90 cm², plus particulièrement d'au plus 80 cm², plus particulièrement d'au plus 70 cm², plus particulièrement d'au plus 60 cm².

17. Couverture opératoire (10) selon l'une quelconque des revendications précédentes, **caractérisée en ce que** la couverture opératoire (10) est un champ opératoire pour l'oeil, un champ opératoire pour le nez, un champ opératoire pour l'oreille, un champ opératoire pour le talon, un champ opératoire pour le poignet ou un champ opératoire pour la cheville.

18. Couverture opératoire (10) selon l'une quelconque des revendications précédentes, **caractérisée en ce que** l'élément matériau de protection (38) comprend une première section d'élément matériau de protection (46) et une deuxième section d'élément matériau de protection (48), la première section d'élément matériau de protection (46) étant pliable le long de la deuxième ligne d'affaiblissement (40) dans la direction de la deuxième section d'élément matériau de protection (48), et la première section d'élément matériau de protection (46) étant utilisable dans cette configuration en tant qu'auxiliaire d'application (60), notamment en tant que moyen de manipulation (62).
